# EUROPEAN PATENT APPLICATION

(11) **EP 3 756 901 A1**
(43) Date of publication of application: **30.12.2020**
(21) Application number: 20182495.0
(22) Date of filing: 26.06.2020
(51) Int. Cl.: B41M 3/14, G07D 7/14, B42D 25/465, B41M 7/00, C12Q 1/68

(54) **METHOD FOR IDENTIFYING AN ARTICLE**

(30) Priority: 26.06.2019 IT 201900010203
(71) Applicant: ARIES S.r.l., 35132 Padova (PD) (IT)
(72) Inventor: BENEDETTI, Alvise, 30123 Venezia (VE) (IT); CRETAIO, Erica, 35036 Montegrotto Terme (PD) (IT); DE TONI, Alessandro, 35143 Padova (PD) (IT); GAETANI, Chiara, 20822 Seveso (MB) (IT); GOTTARDO, Sara, 30010 Camponogara (VE) (IT)
(74) Representative: Caldon, Giuliano

(57) **Abstract**

Method for identifying articles, which provides for: a step of arranging an article provided with a support (1) of hydrophilic material; a step of localized deposition, on a specific application zone (3) of the support (1), of an isolation layer (2) of hydrophobic and transparent material; and after such deposition step, a step of application, on the isolation layer (2), of a molecular marker (4) of hydrophilic nature. The deposition of the isolation layer is obtained by means of application of a mixture comprising a resin present in a percentage substantially comprised between 0.5 and 15 % w/V and selected between a group constituted by acrylic resins, aliphatic resins, aldehyde resins, silicone resins.

## Description

### Field of application

The present invention regards a method for identifying an article.

The method, object of the present invention, is intended to be advantageously employed for identifying articles made of hydrophilic material, both plant and animal (such as for example documents made of paper, wood, fabric, hide/leather, parchment etc.), by means of the application of molecular markers of hydrophilic nature. For example, the present method is particularly suitable for being employed in anti-counterfeiting field, specifically for historic documents or works of art.

### State of the art

There is the particular need in various fields, such as in the field of preservation of historic-art documentation or anti-counterfeiting documentation, to be able to identify and recognize an article so as to recognize the authenticity thereof or to prevent the smuggling thereof. In particular, especially in the scope of historical documentation, it is requested to be able to identify documents made of hydrophilic material, such as materials of plant nature (such as paper material, fabrics, clothes, etc.) or of animal nature (such as parchments, etc.).

It is known in the state of the art to apply molecular markers (such as fluorophores, DNA molecules) to the articles to be recognized, which define a specific code associated with the article to which they are applied and which allow the identification thereof. Such markers generally have hydrophilic nature and are generally applied to the article immersed within a carrier liquid. For example, known in the pharmaceutical field are methods which provide for mixing the markers with an ink before printing, in a manner such that it is possible to identify the product on whose package the ink is subsequently printed. In particular, known from patent application WO 2014/059061 A1 is a method for authenticating an object which provides for covering the latter with a polymer solution which incorporates a molecular marker at its interior.

The patent application US 2009/0286250 A1 describes another authentication method of known type which provides for the use of a cyanoacrylic solution containing a hydrosoluble molecular marker.

This method for identifying articles has not proven suitable for being applied to documents made of hydrophilic material, since the markers, also being of hydrophilic nature, tend to be absorbed by the support material and to be dispersed within the latter, making it difficult if not impossible to detect the marker in order to execute the identification.

In addition, if the identification requires picking up or drawing the marker from the document (for example in order to execute the necessary laboratory analyses), it is necessary to remove part of the document, rendering such method invasive or destructive and hence unsuitable for being applied on documents of historical character or of artistic value.

In addition, the known techniques for the identification by means of molecular markers are unable to ensure a suitable invisibility of the zone treated for the application of the marker, further rendering such techniques unsuitable for application on documents of historical/artistic character.

Known at the state of the art are numerous chemical compositions employed for making impermeable or hydrophobic coatings, as is described for example in the patents US 2015/0030833, EP 3055371, CN 106867405, CN 106366912. In addition, the patent application EP 0925955 A1 describes a hydrophilic coating layer applied on a printing support so as to facilitate the absorption and the resistance of the ink.

Nevertheless, such compositions are not adapted to be employed in an efficient manner due to the abovementioned identification methods, in particular not allowing the assurance of suitable characteristics in terms of invisibility and of document alteration.

### Presentation of the invention

In this situation, the problem underlying the present invention is that of providing a method for identifying an article, which substantially maintains unchanged the physical and optical properties of the article made of materials of hydrophilic nature in the application zone of the marking.

Another object of the present invention is to provide a method for identifying an article which ensures a substantial transparency and a durable stability over time of the application, even in conditions that are different from standard environmental conditions.

Another object of the present invention is to provide a method for identifying an article which is applicable on articles composed of different types of hydrophilic materials (such as paper, fabrics, parchments, hide/leather, etc.).

Another object of the present invention is to provide a method for identifying an article which allows a precise confinement of the markers in the application zone.

Another object of the present invention is to provide a method for identifying an article which allows applying the marking substantially on any zone of the article.

Another object of the present invention is to provide a method for identifying an article which allows recovering the marker from the article without damaging the latter. Another object of the present invention is to provide a method for identifying an article which allows being applicable also to pre-existing articles, even after the production step.

### Brief description of the drawings

The technical characteristics of the invention, according to the aforesaid objects, can be clearly seen in the content of the below-reported claims and the advantages thereof will be more evident in the following detailed description, made with reference to the examples illustrated in the enclosed figures, in which:
- figures 1A-C show an example of several operating steps of the method for identifying an article, object of the present invention;
- figures 2A and 2B show two corresponding images for the detection of a marker (constituted by a fluorophore) executed, respectively, on the article to be identified and after the drawing of the marker from the article itself;
- figure 3 shows a graph which represents an absorption spectrum of a marker constituted by a fluorophore analyzed at the concentration of deposition on the article and after the recovery of such fluorophore;
- figure 4A reports a series of images of two support samples at different times after the deposition of an isolation layer, in accordance with the method that is the object of the present invention;
- figure 4B shows a graph which depicts the values of the color difference ΔE calculated on multiple support samples following the application of the isolation layer;
- figures 5A and 5B respectively show a series of images and a graph relative to the behavior of a water drop (analyzed by means of contact angle) with a hydrophilic material of the support of the article in the absence of or in the presence of the isolation layer;
- figure 5C shows a sheet relative to absorption times of hydrophilic materials of the support of the article in the absence of or in the presence of the isolation layer;
- figure 6 shows a graph relative to colorimetric test results of the isolation layer during an aging process of the support of the article;
- figures 7A and 7B respectively show a series of images and a respective graph relative to the diffusion of a hydrophilic molecule on the isolation layer;
- figures 8A-C, 9A-C and 10A-C report images, values of the contact angle and absorption resistance obtained by means of tests carried out on different types of supports with different exemplifying embodiments of the isolation layer.

### Detailed description

The method for identifying an article, object of the present invention, is intended to be advantageously employed for identifying articles made of hydrophilic material, both plant and animal (such as documents made of paper, fabric, parchment, etc.) by means of the application of molecular markers of hydrophilic nature. In particular, the present method is particularly suitable for being employed in articles constituted by antique documents, of historical and artistic character, which require that the application of the method does not leave any visible trace thereon that would involve a degradation of the article.

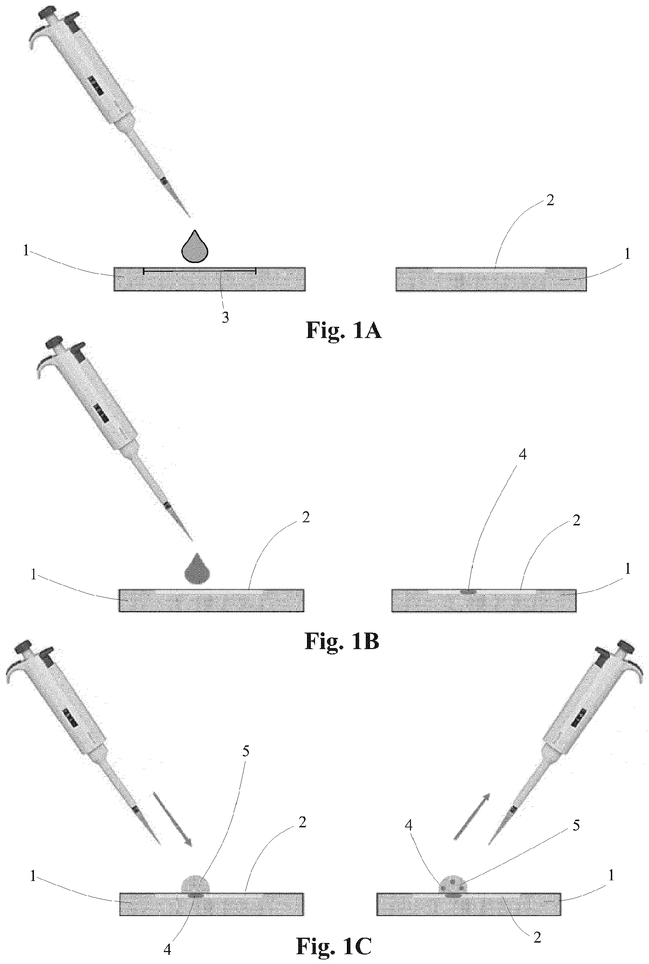

With reference to the example of figures 1A-C, the present method comprises a step of arranging at least one article to be identified (such as a paper document, a painting, etc.). Such article is provided with at least one support 1 of hydrophilic material, which can for example have plant or animal nature.

In particular, by "hydrophilicity" it is intended a property of the material of being similar to water and to polar solvents, since it is provided with a high number of hydrophilic surface groups (such as the -OH group) which thermodynamically facilitate the formation of hydrogen bonds. Consequently, when such material type comes into contact with water, aqueous solutions or polar solvents, the drops tend to be expanded according to two modes: either by penetrating into the thickness of the material, or by diffusing on the surface thereof.

For example, the hydrophilic material of the support 1 of the article can comprise paper material (such as cellulose paper, rice paper, cotton/rag paper), parchments, fabrics/cloths, hide/leather, wood, etc. The article to be identified can for example be constituted by a book, a canvas, a painting, etc.

With reference to the example of figure 1A, the present method comprises a step of localized deposition, on the support 1 of the article, of an isolation layer 2, of hydrophobic and transparent material.

In particular, the isolation layer 2 is deposited on a specific application zone 3 of the support 1, positioned for example in a predefined zone of the support 1 (such as a specific area of a page), and such zone is preferably known only by the subject who executes the method, in a manner such to allow the identification thereof in order to identify the article, as described hereinbelow.

Preferably, the application zone 3 of the support 1 (on which the isolation layer 2 is deposited) has width substantially comprised between 0.5 and 1 cm, e.g. with substantially circular shape.

According to the invention, the step of deposition of the isolation layer 2 is executed by means of the application of a mixture comprising at least one solvent and at least one resin.

Advantageously, such mixture is applied in the liquid or semi-liquid state on the support 1, in a specific quantity, preferably with volume substantially comprised between 0.2 and 5 µL.

In particular, the mixture is applied on the support 1 by means of suitable instruments of known type, such as a syringe, a micro-pipette, or an automated dispensing nozzle (for example in industrial applications).

The resin, which is dissolved within the solvent of the mixture applied to the support 1, advantageously has molecular weight substantially between 200 and 2000 g/mol and is selected between the group constituted by acrylic resins, aliphatic resins, aldehyde resins, silicone resins.

The aforesaid resin is contained in the solvent in a percentage substantially comprised between 0.5 and 15 % w/V, calculated as ratio between weight of the resin over a volume of the solvent.

In particular, the measurement unit "% w/V by weight of the resin over a volume of the solvent" expresses the weight in grams of the resin in a volume of 100 mL of solvent, thus indicating the percentage ratio between the weight in grams of the resin and the volume in milliliters of the solvent.

More generally, by "% w/V by weight of solute over a volume of solvent" it is intended the weight in grams of the solute in a volume of 100 mL of solvent.

Preferably, in the case of acrylic or aldehyde resin, this is present in the solvent in a percentage substantially comprised between 1 and 5 % w/V.

Suitably, in the case of resin of aliphatic type, this is present in the solvent in a percentage substantially comprised between 1 and 15 % w/V, and preferably between 2.5 and 10 % w/V.

As discussed in detail hereinbelow, the deposition of the mixture with the claimed resin allows modifying, in a selective and localized manner, the properties of the support 1 to be bonded with water (or aqueous solutions), rendering the application zone 3 of the support 1 hydrophobic. In particular, the claimed mixture allows determining an exclusive modification of only the hydrophilicity of the support 1 in the application zone 3, without generating substantial modifications of any physical or optical property of the material of the support 1, in particular of the color.

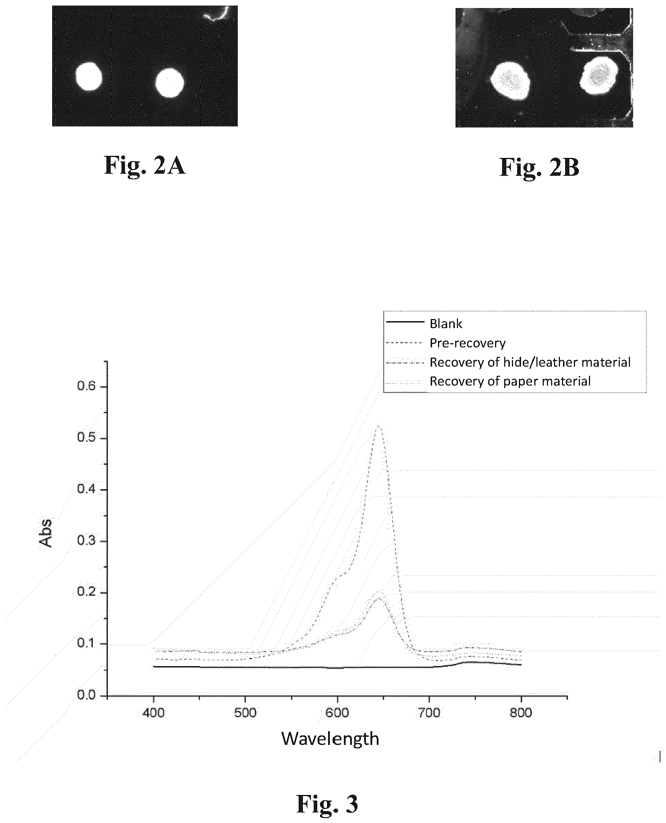

With reference to the example of figure 2B, the present method comprises, after the step of deposition of the isolation layer 2, a step of application, on such isolation layer 2, of at least one molecular marker 4 of hydrophilic nature.

Following the hydrophobic nature of the isolation layer 2 (determined in particular by the claimed resin that constitutes it), the molecular marker 4 is not diffused within the hydrophilic material of the support 1, but remains localized in the application zone 3 on which the isolation layer 2 is deposited.

For example, such molecular marker 4 can be applied with a micro-syringe, or other appropriate instrumentation.

The molecular marker 4 can be of any type known in the state of the art as a function of the particular application situation and, preferably, is selected from the group comprising nucleic acids, fluorophores, organic biomolecules and compounds.

Suitably, the molecular markers 4 can be selected as a function of the requested security level and of the speed with which it is necessary to authenticate the article. For example, it is possible to employ as markers: water-soluble fluorophores, fluorescent DNA, and proteins that are specifically bonded to other proteins by means of specific bonds. Advantageously, the present method comprises a step of authentication of the article, by means of analysis of the molecular marker 4 applied on the isolation layer 2. In particular, in such authentication step, the molecular marker 4 is subjected to a process, which depends on the type of marker, in order to detect the code associated with such marker, which can be given for example by a DNA sequence (in the case of nucleic acids), or the emission of a specific color or wavelength if subjected to specific electromagnetic rays.

The analysis of the molecular marker 4 for the authentication of the article can be executed on site or in a different venue (such as a laboratory) as a function of the property of the marker 4 itself.

Advantageously, with reference to figure 1C, the aforesaid authentication step provides for drawing the molecular marker 4 from the isolation layer 2 so as to subject it to an encoding analysis, by means of suitable instruments, for example in laboratory settings. In particular, the step of application of the molecular marker 4, executed after the deposition of the isolation layer 2, allows recovering the molecular marker 4 without damaging the article.

For example, the drawing of the molecular marker 4 occurs by means of application of at least one recovery solution 5 on the isolation layer 2 (e.g. by means of a micro-syringe), in a manner such that at least part of the molecular marker 4 is dissolved in such recovery solution 5. Subsequently, the recovery solution 5 with the molecular marker 4 dissolved at its interior is drawn from the isolation layer 2, for example by suctioning it by means of the same micro-syringe employed for its application.

In accordance with an embodiment variant, the authentication step can provide for, in addition to or as an alternative to drawing the molecular marker 4, an analysis of the latter when this is on the isolation layer 2 applied on the article, for example in the case of fluorescent markers, or markers recognizable following the application of specific electromagnetic waves.

Advantageously, the mixture for attaining the isolation layer 2 comprises silica nanoparticles, dissolved in the solvent preferably in a percentage substantially comprised between 0.5 and 1 % w/V. The addition of such silica nanoparticles determines an effect of opacifying and color subtraction diminution, improving the invisibility of the isolation layer 2. The silica nanoparticles also allow improving the containment of the molecules of the molecular marker 4, also facilitating the recovery of the latter in the authentication step.

Several particular exemplifying applications of the present method, which employ different marker types, are described hereinbelow.

A first application example provides for the use of a fluorophore as molecular marker 4. In such case, the presence of the molecular marker 4 can be monitored both on the support 1 of the article on which it has been applied, and after it has been recovered from the article (and then analyzed in a laboratory with the use of a scanner). Such operation is made possible due to the presence of the hydrophobic isolation layer 2, which makes possible the localized application of the molecular marker 4 and the subsequent detection. Figure 2A shows the fluorophore positioned on an article constituted by a paper document superimposed on the hydrophobic isolation layer 2. The diffusion of the fluorophore between the fibers of the paper is limited by the arrangement of the isolation layer 2, making possible the subsequent recovery of the marker 4 (fluorophore). The analysis of the marker 4 recovered with the use of a scanner is shown in figure 2B, in which the intensity of the emission is comparable to that of figure 2A, to indicate the recovery of a considerable part of the deposited fluorophore.

A second application example provides for using, as molecular marker 4, a molecule recognizable with a spectroscopic analysis. In this case, the molecule has an absorption spectrum which corresponds with a fingerprint, which allows uniquely recognizing it. Hence, if this molecule is deposited on an article of interest and then subsequently recovered it is possible to uniquely analyze and recognize it. Figure 3 shows an absorption spectrum of a fluorophore excited at the suitable wavelength, analyzed at the concentration of deposition on the isolation layer 2 and after the recovery of the molecule.

A third application example provides for the use, as molecular marker 4, of a DNA-based marker of different length. Since DNA is a hydrophilic molecule, which is thus dissolved in water, it is necessary to apply the isolation layer 2 in order to prevent the dispersion of the molecules in the hydrophilic material of the support 1 (e.g. in the paper cellulose fibers), which would make the recovery thereof impossible. In addition, the DNA, in the form of different-length filaments, is a code known to the subject who deposits it and for this reason a high level of security is assured. Once the DNA-based marker has been recovered, it possible to analyze it by hybridizing the probe with the complementary filament (probe). The probe must be paired with a molecule (such as fluorophores, proteins, enzymes, etc.) that is capable of emitting a signal to demonstrate that probe recognition has occurred. In order to identify the DNA molecule of the molecular marker 4, it is possible to employ two methods. A first method provides for executing a pre-analysis of the DNA present in the sample of the marker 4 recovered by means of nano-drop spectrophotometer. In this manner, by measuring the absorbance at the specific wavelengths, it is possible to have an indication on the DNA content of the sample. A second method provides for analyzing the filaments of DNA (probe) on a micro-array suitably prepared according to procedures that are per se known in reference field. A third method provides for analyzing the DNA by means of polymerase chain reaction technique (PCR or rt-PCR) according to procedures per se known in the reference field.

Several examples of the mixture employed for obtaining the isolation layer 2 according to the present method are described hereinbelow, so as to demonstrate the obtained characteristics which allow attaining the objects of the present invention.

### Transparency of the isolation layer

Advantageously, the time necessary for obtaining a complete drying of the isolation layer 2 after the deposition step was experimentally determined and resulted substantially comprised between about 30 and 120 minutes, in particular varying as a function of the level of hydrophilicity of the material of the support 1.

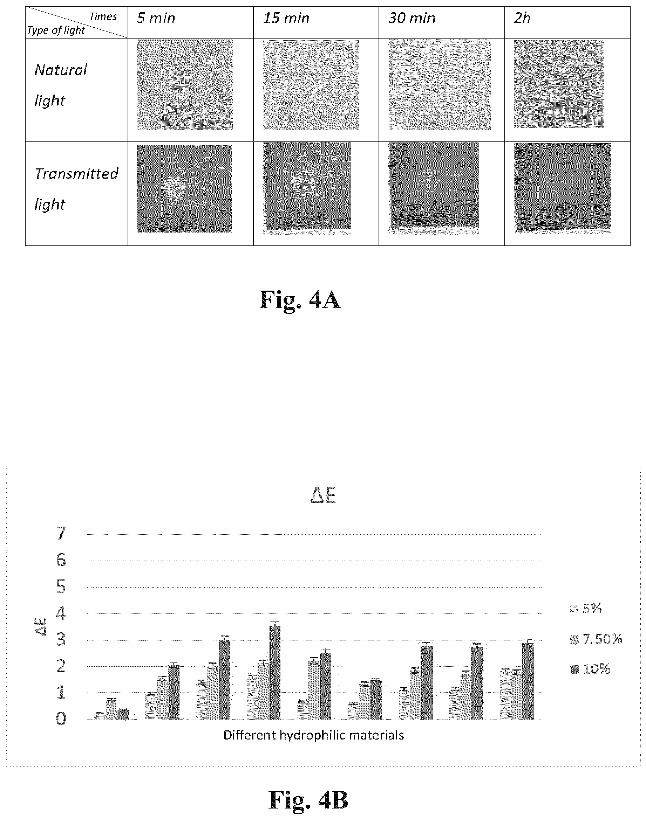

During the drying of the isolation layer 2, the disappearance of the halo caused by such isolation layer 2 was monitored, with the use of an overhead projector. At the end of the complete drying of the isolation layer 2, the latter resulted substantially invisible both in natural light and in transmitted light, as results from the example illustrated in the sheet of figure 4A, which reports several photographs of a support sample 1 made of very hydrophilic paper executed at different times after the deposition of an isolation layer 2 obtained by means of a mixture constituted by aliphatic resin dissolved in white spirit in a percentage of 3% w/V.

The transparency of the isolation layer 2 was also evaluated by means of colorimetric analysis, through the use of colorimeters (in particular the model "Spectra Magic Konica Minolta") capable of providing analysis of pigments and dyes. In such analyses, the colorimeter was employed for evaluating possible variations of the color of the hydrophilic material of the support 1 in the application zone 3 before and after the deposition of the isolation layer 2, so as to verify if the added isolation layer 2 can be visibly perceived and if it has an impact regarding color.

The parameter taken under consideration was the color difference ΔE, which, as is known, represents the distance in the chromatic space CIE L*a*b* between two colors and therefore is a value that indicates the color variation over a selected analysis area. The color difference was calculated between the color of the article in the area of application 3 after the deposition of the isolation layer 2 with respect to an area of the support 1 where the isolation layer 2 was not applied.

The graph reported in figure 4B depicts the calculated ΔE values on support samples 1 (constituted by: hydrophilic historical paper, modern printer paper, wood, parchment, hide/leather) following the application of a mixture comprising aliphatic resin dissolved in white spirit in three different concentrations (5% w/V, 7.5% w/V, 10% w/V). As results from the of figure 4B, the value of ΔE increases as a function of the concentration of the resin, without ever exceeding a value of 3.5. In the case of the resin at the lower concentration (5% w/V), ΔE almost never exceeds the value of 1.5, increasing slightly for the resin at the intermediate concentration (7% w/V). With the greater resin concentration (10% w/V), ΔE never exceeds the value of 4.

It is known that the values of ΔE lower than 5, in this application field, correspond to color variations that are invisible to the naked eye, confirming that the application zone 3 on which the isolation layer 2 is deposited cannot be visibly identified. Therefore, the physical/optical properties of the hydrophilic material of the support 1 of the article were not modified by the addition of the isolation layer 2.

### Hydrophobicity of the isolation layer

In order to determine the hydrophobicity of the isolation layer 2 on the hydrophobic material of the support 1 of the article, two parameters were evaluated relative to the interaction between the material and water:
1) the hydrophilicity was evaluated by measuring the contact angle between the surface of the isolation layer 2 and a 5 µL deionized water drop. The threshold value that defines this chemical characteristic is 90°: below this value, a surface is considered hydrophilic, while above this value it is considered hydrophobic.
2) the capacity of the support with the isolation layer 2 to resist the absorption of water drops was evaluated.

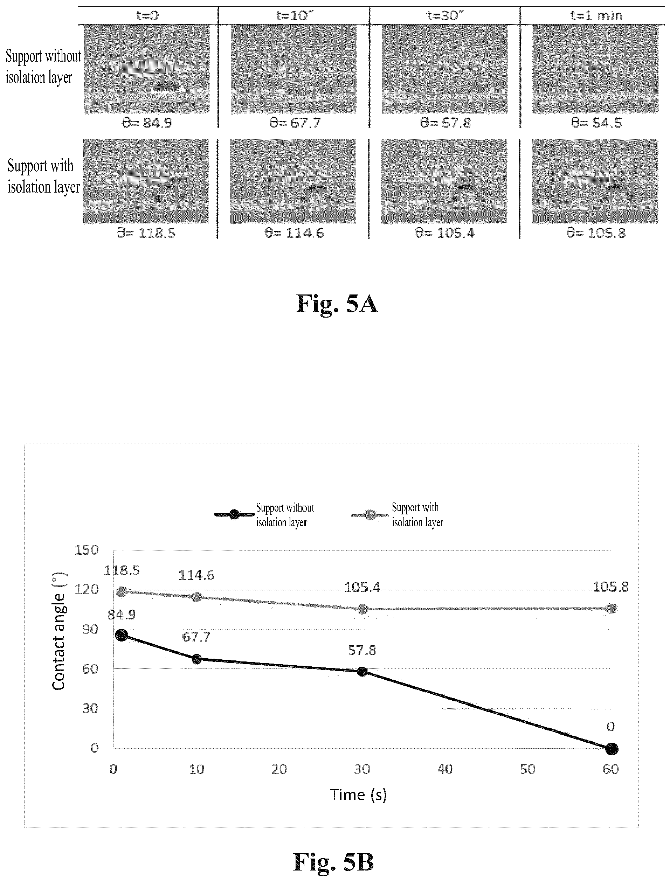

In the images and in the graph of figures 5A and 5B, an example is presented of how a 5 µL water drop behaves in contact with a hydrophilic material (composed of a paper) in the absence of or in the presence of the isolation layer 2 constituted by aliphatic resin dissolved in butyl acetate in a percentage of 10% w/V.

As is inferred from the photographs of figure 5A, which monitor the behavior of the drop, it is observed that if the isolation layer 2 is absent (upper line of figure 5A), the water drop is totally absorbed: the contact angle is below the threshold value of 90° and decreases over time up to the disappearance of the drop due to the absorption thereof. If the isolation layer 2 is present on the material (lower line of figure 5A), the contact angle increases by at least 33°, exceeding the threshold value of 90° and rendering the application zone 3 hydrophobic.

The average value of the contact angle, measured after the deposition of the isolation layer 2 on a sample of about thirty materials, resulted 110°±5.

The hydrophilicity and the contact angle of the surface is also a function of the roughness of the surface, which strongly affects the water drop absorption process. Notwithstanding the non-uniformity of the analyzed hydrophilic materials employed as support 1, also in terms of surface roughness, the recorded values are always greater than 90°, the limit for defining a material as hydrophobic or hydrophobic.

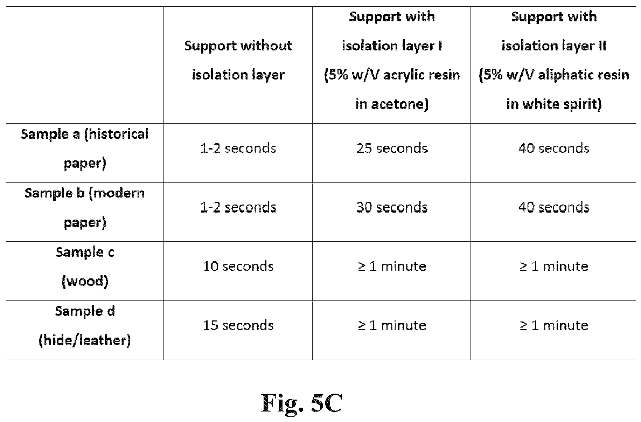

In addition, the presence of the isolation layer 2 considerably delays the absorption, also maintaining the value of the contact angle nearly unchanged. Such behavior is always associated with an increase of the water absorption resistance, as demonstrated by the values of the absorption times reported in the sheet of figure 5C, which are related to tests made with a 5 µL water drop, for an observation time of one minute, on four samples of hydrophilic materials (historical paper, modern paper, wood, hide/leather) with a hydrophobic layer containing acrylic resin dissolved in acetone in a percentage of 5% w/V (isolation layer I) and with a hydrophobic layer containing aliphatic resin dissolved in white spirit in a percentage of 5% w/V (isolation layer II).

As shown by the test results, the presence of the isolation layer 2 considerably increases the absorption resistance of the materials of the support 1. For the super-hydrophilic materials (samples a and b), the isolation layer 2 with the acrylic resin (isolation layer I) increases the drop seal by 300%, passing from 1-2 seconds to 25-30 seconds. The isolation layer 2 with aliphatic resin (isolation layer II) determines an over 100% increase, passing from 30 to 40 seconds.

With regard to the hydrophilic materials (samples c and d), without the isolation layer 2 these resist the water absorption for 10-15 seconds, while an increase of the water resistance is recorded that is greater than or equal to 60 seconds with the isolation layers 2 with both resins.

### Stability over time of the isolation layer

The stability over time of the isolation layer 2 was monitored and evaluated by observing the behavior thereof and using, as hydrophilic material of the support 1, samples of hydrophilic paper. The samples were subjected to an accelerated artificial aging treatment, by subjecting the samples to 80 °C at an RH of 65% for 28 days.

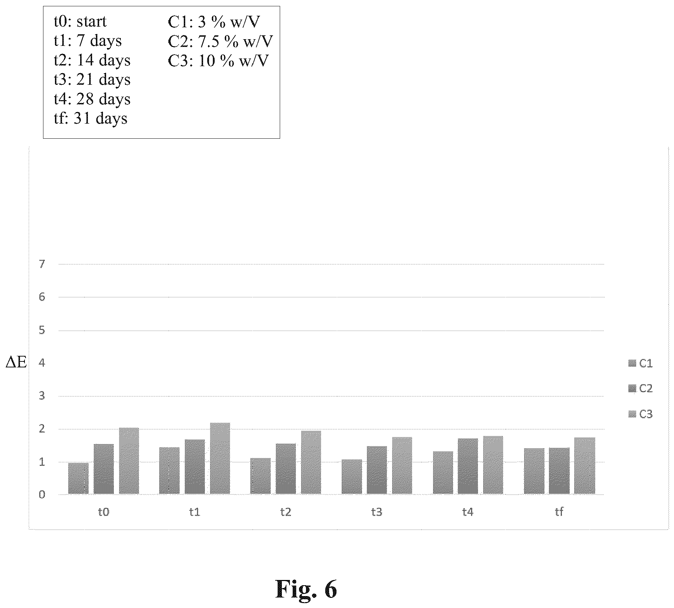

The graph of figure 6 shows the results of colorimetric tests adapted to verify the color difference between the application zone 3 of the hydrophilic material before and after the application of the isolation layer 2 containing aliphatic resin dissolved in white spirit in three different percentages (3% w/V; 7.5% w/V; 10% w/V), detected at multiple times during the aging treatment: as can be detected, the values of the measured parameter ΔE remain below the threshold value of 5.

### Capacity of containing the marker determined by the isolation layer

The application of the isolation layer 2 on the hydrophilic material of the support 1 of the article allows applying the hydrophilic molecules of the molecular marker 4, preventing the diffusion of hydrophilic material, e.g. in the cellulose material fibers.

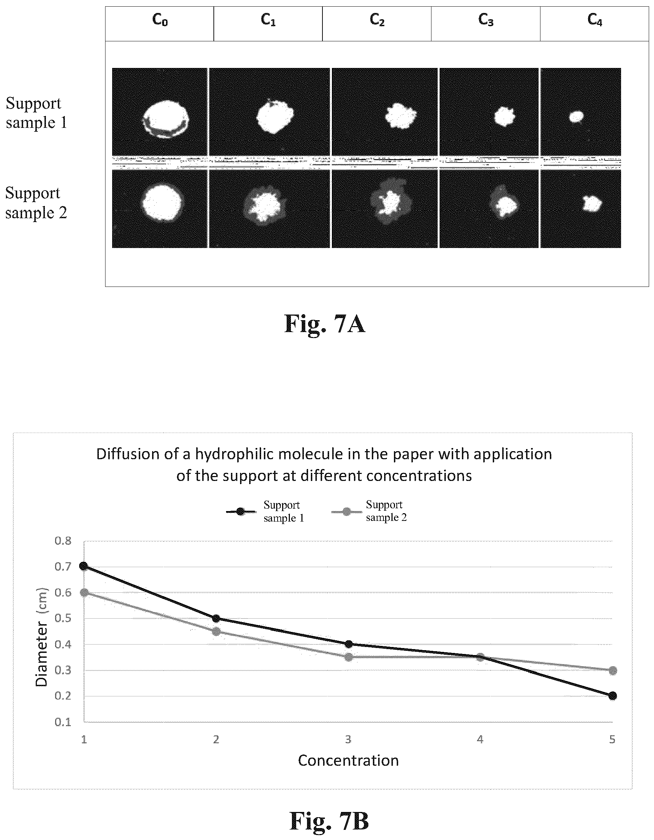

The images and the graph of figures 7A and 7B show the diffusion of a hydrophilic molecule on two different samples of hydrophilic paper material (hydrophilic historical paper and modern paper) in the application zone 3 on the isolation layer 2 containing aliphatic resin dissolved in butyl acetate in five different percentages (comprised between 0.5-15% w/V). As demonstrated by such results, in addition to ensuring transparency and hydrophobicity, the isolation layer 2 offers the possibility of limiting the diffusion of hydrophilic molecules of the molecular marker 4, increasing the containment capacity in particular as a function of the resin concentration.

### Specific examples

Table 1 hereinbelow reports several particular non-limiting examples of the composition of the mixture employed for obtaining the isolation layer.

**Table 1**

| **Example** | **Resin** | **Solvent** | **Resin concentration** |
|---|---|---|---|
| Example 1 | Acrylic resin, copolymer produced from ethylacrylate and methacrylate molecules | Acetone | 1-5% w/V |
| Example 2 | Aliphatic resin obtained from the polymerization of vinyl-toluene and alphamethyl-styrene | White spirit | 0.5-15% w/V |
| Example 3 | Urea-aldehyde resin, produced with the condensation of low-molecular-weight aliphatic aldehydes with urea | White spirit, cyclohexane | 1-5% w/V |

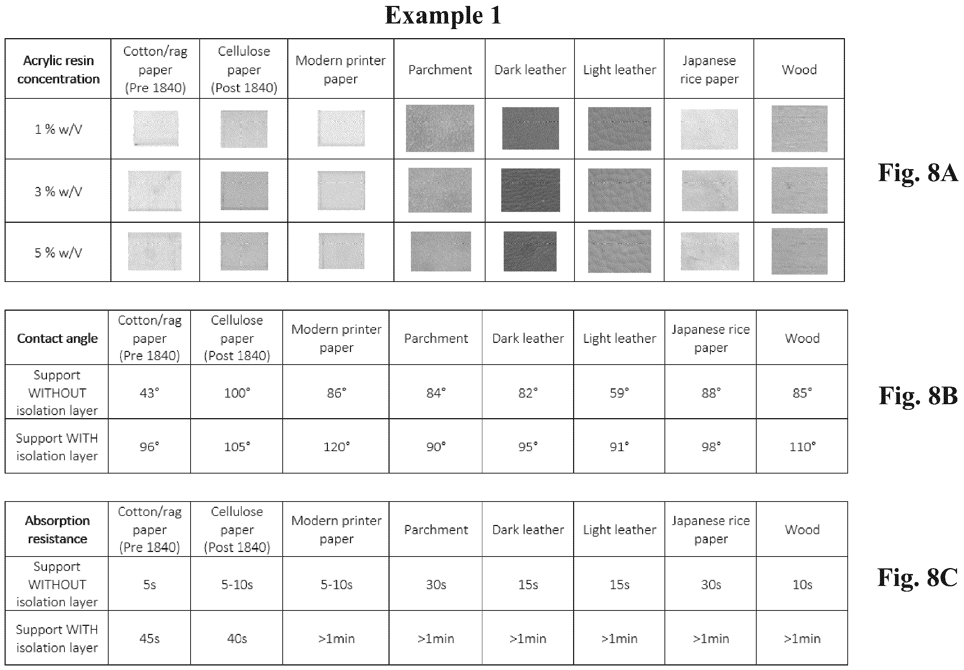

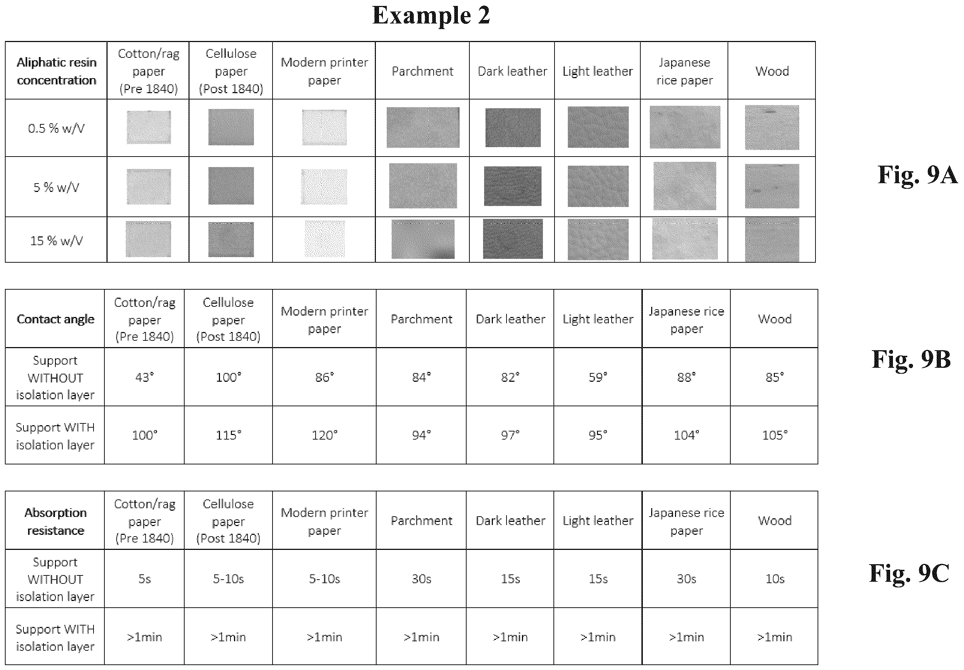

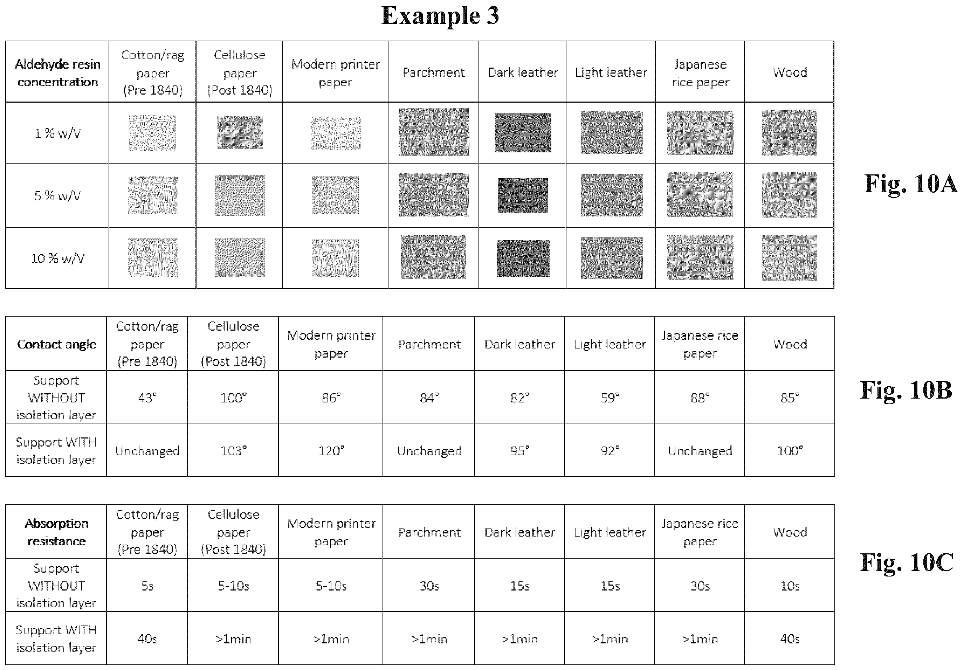

Figures 8A-C, 9A-C and 10A-C report images, contact angle and absorption resistance values obtained by means of tests carried out on different types of supports 1 (indicated in the figures) with the above-reported mixtures with different concentrations of the respective resins (also reported in the figures).

The images of figures 8A, 9A and 10A indicate that the isolation layer 2 is substantially invisible over all the samples of hydrophilic material to which it has been applied.

The values of the contact angles are comparable for all the concentrations of each resin and, therefore, these are reported only once for each example. In particular, also in low resin concentration, the isolation layer 2 provides the same level of hydrophobicity to the hydrophilic material. The values presented in the sheets of figures 8B-C, 9B-C and 10B-C are recorded one minute after the deposition of a 5 µL water drop on the isolation layer 2 in the application zone 3, demonstrating not only an increase of the hydrophobicity of the material, but also an increase of the water absorption resistance.

From the results of the above-illustrated examples, overall it results that the resins of all the examples have at least one concentration condition in which the isolation layer 2 is invisible and provides an increase of the local hydrophobicity of the hydrophilic material on which it has been deposited. Specifically, the acrylic resin (example 1) and the aldehyde resin (example 3) determine good invisibility characteristics, in particular in the concentration range lower than 5% w/V. The aliphatic resin (example 2) meets the invisibility requirement for a particularly extensive range of concentrations, substantially comprised between 1 and 15% w/V.

With regard to the hydrophobicity, the best performance is provided by the aliphatic resin, with an average increase of the contact angle of 25°, with respect to 20° of the acrylic resin and 11° of the aldehyde resin.

A positive effect that all the tested resins have is the capacity of considerably limiting the water absorption. Indeed, if in the absence of the isolation layer 2 the super-hydrophilic materials absorb one water drop in less than 10 seconds, this time is delayed up to over a minute in the presence of the isolation layer 2.

The invention thus conceived therefore attains the pre-established objects.

In particular, the application of the isolation layer - transparent, hydrophobic and stable over time - on the hydrophilic material of the article allows rendering the latter locally hydrophobic without modifying the optical characteristics thereof. In addition, the isolation layer can be positioned, as a function of requirements, in any zone of the article since it is able to locally modify the article's affinity with water, being maintained transparent and permanent over time.

In addition, since the isolation layer is transparent in natural and transmitted light, and hence not easily identifiable, once deposited it can only be located again by the person who deposited it, by means of the use of suitable spatial references, ensuring a high level of security.

## Claims

1. Method for identifying articles, which provides for:
- a step of arranging at least one article provided with at least one support (1) of hydrophilic material;
- a step of localized deposition, on a specific application zone (3) of said support (1), of an isolation layer (2) of hydrophobic and transparent material, by means of application of a mixture comprising at least:
- a solvent;
- a resin dissolved in said solvent in a percentage substantially comprised between 0.5 and 15 % w/V by weight of said resin over a volume of said solvent; wherein said resin is selected from a group constituted by acrylic resins, aliphatic resins, aldehyde resins, silicone resins;
- after said step of deposition of said isolation layer (2), a step of application, on said isolation layer (2), of at least one molecular marker (4) of hydrophilic nature.

2. Method according to claim 1, **characterized in that** said at least one resin has molecular weight substantially between 200 and 2000 g/mol.

3. Method according to claim 1 or 2, **characterized in that**, in said step of deposition, a volume of said mixture is applied substantially comprised between 0.2 and 5 µL.

4. Method according to any one of the preceding claims, **characterized in that**, in said step of deposition, the application zone (3) of said support (1) has width substantially comprised between 0.5 and 1 cm.

5. Method according to any one of the preceding claims, **characterized in that** said resin is an aliphatic resin present in said solvent in a percentage substantially comprised between 1 and 15 % w/V.

6. Method according to any one of the preceding claims, **characterized in that** said solvent is selected from a group constituted by acetone, ethanol, petroleum essence, white-spirit, cyclohexane.

7. Method according to any one of the preceding claims, **characterized in that** said mixture comprises silica nanoparticles.

8. Method according to any one of the preceding claims, **characterized in that** it comprises an authentication step, by means of a process of analysis of said molecular marker (4).

9. Method according to claim 8, **characterized in that** said authentication step provides for drawing said molecular marker (4) from said isolation layer (2), by means of application of at least one recovery solvent (5) on said isolation layer (2), said recovery solvent (5) causing the dissolving therein of at least part of said molecular marker (4), and subsequent drawing of at least part of said recovery solvent (5) with at least part of said molecular marker (4) dissolved therein.

10. Method according to any one of the preceding claims, **characterized in that** said molecular marker (4) is selected from a group comprising genetic markers, fluorophores, organic dyes.
